# EUROPEAN PATENT SPECIFICATION

(11) **EP 3 113 071 B1**
(45) Date of publication and mention of the grant of the patent: **05.02.2025**
(21) Application number: 16166264.8
(22) Date of filing: 20.04.2016
(51) Int. Cl.: G06V 40/18, G06V 40/19

(54) **METHOD AND DEVICE FOR ACQUIRING IRIS IMAGE**
VERFAHREN UND VORRICHTUNG ZUR ERFASSUNG VON IRISBILDERN
PROCÉDÉ ET DISPOSITIF POUR ACQUÉRIR UNE IMAGE DE L'IRIS

(30) Priority: 30.06.2015 CN 201510373998
(43) Date of publication of application: 04.01.2017
(73) Proprietor: Xiaomi Inc., Beijing 100085 (CN)
(72) Inventor: ZHANG, Tao, 100085 Haidian District, Beijing (CN); LONG, Fei, 100085 Haidian District, Beijing (CN); CHEN, Zhijun, 100085 Haidian District, Beijing (CN)
(74) Representative: Reddie & Grose LLP

(56) References cited:
- US-A1- 2013 089 240
- "Iris Biometrics", vol. 59, 1 January 2013, SPRINGER, ISBN: 978-1-4614-5571-4, ISSN: 1568-2633, article CHRISTIAN RATHGEB ET AL: "Iris Biometrics", pages: 7 - 11, XP055323651
- "Sankara Nethralaya Atlas of Imaging in Ophthalmology", 1 January 2014, JAYPEE, article AMBIKA SELVAKUMAR: "Sankara Nethralaya Atlas of Imaging in Ophthalmology", pages: 21-35,46-48,119-130, XP055323670
- BURGE MARK: "Handbook of Iris Recognition", 1 January 2013, SPRINGER, pages: 157, XP093117247

## Description

### TECHNICAL FIELD

The present disclosure generally relates to the field of image acquisition technology, and more particularly, to a method and a device for acquiring an iris image, and a device for identifying an iris.

### BACKGROUND

At present, techniques for capturing iris images of an individual have been more and more applied to the field of personal identification due to the uniqueness of individual irises. Existing methods for acquiring an image of the iris need be achieved with specialised iris acquisition apparatus. However, during the acquisition of an iris image of a use, the iris acquisition apparatus requires the user to fully cooperate with the apparatus, resulting in an increased difficulty and lower user experience in using the iris acquisition apparatus.

Document US2013089240A1 discloses a portable, hand held iris imaging system for capturing iris images that may be used in biometric identification. The system is constructed using two separate but coupled subsystems. A first subsystem augments the underlying functionality of the second subsystem. The first subsystem uses an iris camera to capture iris images. A tunable optical element positioned between the subject and the iris camera focuses light reflected from the subject's eye onto the iris camera. A controller coordinates the capture of the iris image with the second subsystem. The second subsystem captures face images of the subject, which are provided to a display through a computer. The user interface is overlaid over the face images to provide visual feedback regarding how the system can be properly repositioned to capture iris images. The system has a portable form factor so that it may be easily operated. The system does not include a technique for an efficient computation of the radius of the iris.

### SUMMARY OF THE INVENTION

Embodiments of the present disclosure provide a method and a device for acquiring an iris image and a device for identifying an iris, according to the independent claims, so as to be able to accurately acquire the iris image without high cooperation of a user with an iris acquisition apparatus.

According to a first aspect of embodiments of the present disclosure, there is provided a method for acquiring an iris image. The method is defined in claims 1 to 3.

According to a second aspect of embodiments of the present disclosure, there is provided a mobile phone for acquiring an iris image. The mobile phone is defined in claims 4 to 6.

According to embodiments of the present disclosure, the technical solution may have the following advantageous effects. The shooting parameter for shooting the iris is determined based on the acquired image including the eye site. As the image including the eye site may be acquired by various methods, for example, acquired by a common visible light shooting method. As a result, the above described method may be able to shoot the eye site of a user normally standing without requiring the user to adhere his or her eye to a specific position or to closely cooperate with an iris acquisition apparatus, thereby being able to determine the shooting parameter for shooting the iris. The above described method may be able to accurately acquire the iris image without close cooperation by the user, thus improving the user's experience effect. The invention according to the independent claims allows an efficient computation of the radius of the iris on a mobile phone.

It is to be understood that both the foregoing general description and the following detailed description are illustrative and explanatory only and are not restrictive of the disclosure, within the scope of the independent claims.

### BRIEF DESCRIPTION OF THE DRAWINGS

The accompanying drawings, which are incorporated in and constitute a part of this specification, illustrate embodiments consistent with the disclosure and, together with the description, serve to explain the principles of the disclosure.
Fig. 1 is a flow chart showing a method for acquiring an iris image according to an illustrative embodiment;
Fig. 2 is a block diagram showing a device for acquiring an iris image according to an illustrative embodiment;
Fig. 3 is a block diagram showing another device for acquiring an iris image according to an illustrative embodiment;
Fig. 4 is a block diagram showing a further device for acquiring an iris image according to an illustrative embodiment;
Fig. 5 is a block diagram showing a device for identifying an iris according to an illustrative embodiment; and
Fig. 6 is a block diagram of a device 900 for acquiring an iris image according to an illustrative embodiment.

### DETAILED DESCRIPTION

Reference will now be made in detail to illustrative embodiments, examples of which are illustrated in the accompanying drawings. The following description refers to the accompanying drawings in which the same numbers in different drawings represent the same or similar elements unless otherwise represented. The implementations set forth in the following description of illustrative embodiments do not represent all implementations consistent with the disclosure. Instead, they are merely examples of apparatuses and methods consistent with aspects related to the disclosure as recited in the appended claims.

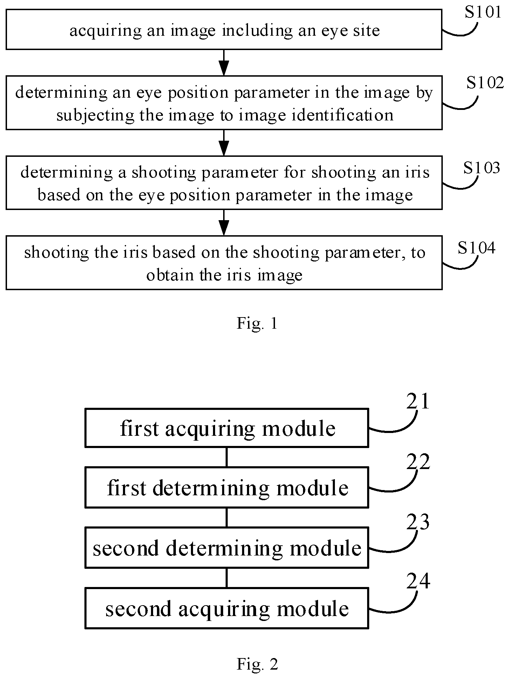

The present disclosure provides in embodiments a method for acquiring an iris image. The method is used to acquire and identify the iris image and used in a device for acquiring the iris image. The method is performed by a mobile phone having an image acquisition or photographic function. As shown in Fig. 1, the method includes the following steps S101 to S104.

In the step S101, an image including an eye site is acquired. In this context, the phrase eye site will be understood as the region of an individual's body where the eye is found, such as the face or a smaller region of the face.

In an embodiment, the step S101 is implemented by shooting the eye site to obtain the image including the eye site. In a practical implementation, after an instruction of acquiring the iris image is received, the AdaBoost classifier may be used to rapidly detect a general area of the eye site, and shoot the eye site within a shooting region once or multiple times, so as to obtain one or more images including the eye site. In such an implementation, a visible light camera may be used for shooting, to obtain the image including the eye site, or any camera enabled device having a photography function.

In the step S102, an eye position parameter in the image is determined by subjecting the image to image recognition or identification.

According to the invention, the step S102 is implemented by determining an eye position in the image by subjecting the image to the image identification; and determining an eye central position and an eyeball radius in the image based on the eye position. That is, the eye position parameter in the image includes the eye central position and the eyeball radius, in other words, a shooting parameter for shooting the iris image can be determined just with such two parameters. The image including the eye site is identified by a radial symmetry transform (RST) method, which may detect a circle rapidly by calculating each point from multiple images to obtain a gradient, and subjecting all points in such a radial direction to cumulative voting reversely such that the most votes are finally cumulated at the eye central position, thereby obtaining the eye position parameter.

In another embodiment, an eye position in the image may be determined by determining a region where the eye is in the image by a self-adaptive AdaBoost algorithm. Based on the eye position, an eye central position and an eye radius in the image is determined by determining the eye central position and the eye radius in the image by radial symmetry transform (RST) within the region where the eye is.

In the step S103, a shooting parameter for shooting an iris is determined based on the eye position parameter in the image.

In an embodiment, the shooting parameter for the camera function, including a focal point position, a focal length, brightness and contrast, and the like, is set based on the eye position parameter obtained in the step S102, to be capable of obtaining a complete and clear iris image with an appropriate size. For example, based on the image including an eye site and acquired by shooting with a visible light camera, after the eye central position is determined, a distance to the eyeball central position is determined as 30 cm and current ambient brightness is determined as 100 cd/m², then after the focal point (the eye central position) is captured, the shooting parameter for shooting the iris may be set as 8× of the focal length, -20 of the brightness and 18 of the contrast.

In the step S 104, the iris is shot based on the shooting parameter, to obtain the iris image.

In an embodiment, a device for shooting the iris may be a near-infrared camera, as a result, the shooting parameter determined in the step S103 may be a near-infrared shooting parameter applicable for the near-infrared camera. Therefore, the step S104 may be implemented by shooting the iris based on the near-infrared shooting parameter to obtain the iris image. The iris image obtained by the near-infrared shooting technique is of an improved clarity.

The method according to embodiments of the present disclosure, the shooting parameter for shooting the iris is determined based on the acquired image including the eye site. As the image including the eye site may be acquired by various methods, for example, acquired by a common visible light shooting method. As a result, the above described method may be able to shoot the eye site of a user normally standing, without requiring the user to adhere his or her eye to a specific position or to closely cooperate with an iris acquisition apparatus, thereby being able to determine the shooting parameter for shooting the iris. The above described method is able to accurately acquire the iris image without close cooperation by the user, thus improving the user's experience.

In an embodiment, when the step S101 is implemented by shooting the eye site to obtain the image including the eye site, the above described method has the following advantageous effects. The shooting parameter for shooting the iris is determined based on the acquired image including the eye site. The image including the eye site may be acquired by a common visible light shooting method. As a result, the image including the eye site can be shot when a user stands normally, instead of adhering his or her eye to a specific position or closely cooperating with a shooting apparatus, thereby being able to determine the shooting parameter for shooting the iris. The above described method is able to accurately acquire the iris image without close cooperation by the user, thus improving the user's experience.

According to a second aspect of embodiments of the present disclosure, corresponding to the above method for acquiring the iris image, there is provided a device for acquiring an iris image. As shown in Fig. 2, the device includes: an first acquiring module 21, configured to acquire an image including an eye site; a first determining module 22, configured to determine an eyeball position parameter in the image by subjecting the image to image identification; a second determining module 23, configured to determine a shooting parameter for shooting an iris based on the eyeball position parameter in the image; and a second acquiring module 24, configured to shoot the iris based on the shooting parameter, to obtain the iris image.

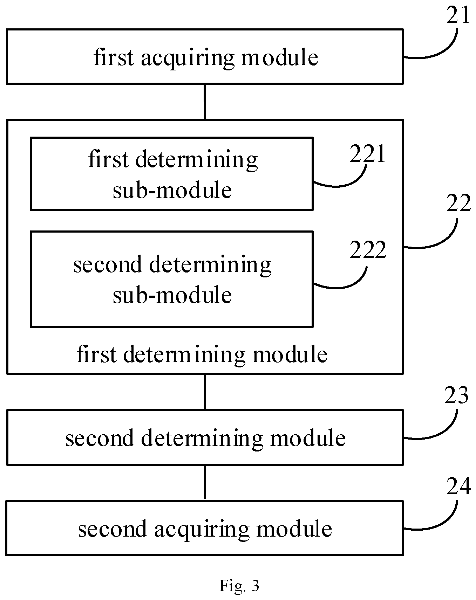

According to the invention, as shown in Fig. 3, the first determining module 22 includes: a first determining sub-module 221, configured to determine an eye position in the image by subjecting the image to the image identification; and a second determining sub-module 222, configured to determine an eye central position and an eye radius in the image based on the eye position.

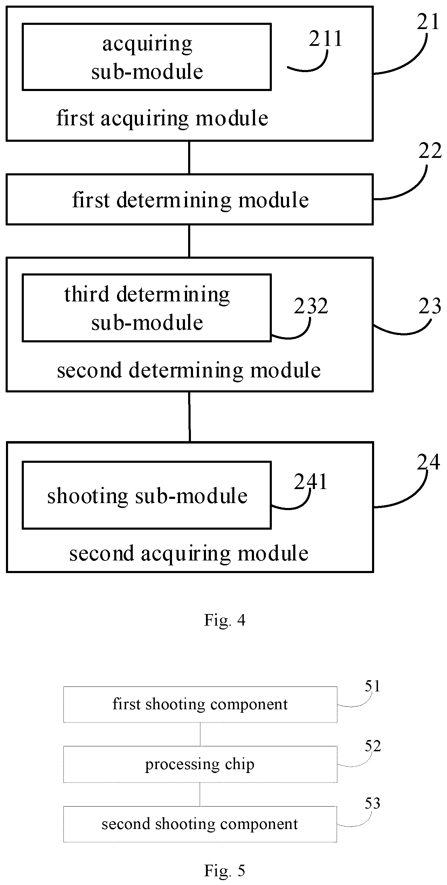

In an embodiment, as shown in Fig. 4, the second determining module 23 may include a third determining sub-module 232, configured to determine a near-infrared shooting parameter for shooting the iris based on the eye position parameter in the image; and the second acquiring module 24 may include a shooting sub-module 241 configured to shoot the iris based on the near-infrared shooting parameter, to obtain the iris image.

In an embodiment, as shown in Fig. 4, the first acquiring module 21 may include: an acquiring sub-module 211, configured to shoot the eye site, so as to obtain the image including the eye site.

In an embodiment, the first determining sub-module 221 is further configured to determine a region where the eye is in the image by a self-adaptive AdaBoost algorithm.

According to the invention, the second determining sub-module 222 is further configured to determine the eye central position and the eye radius in the image by radial symmetry transform (RST) within the region where the eye is.

According to a third aspect of embodiments of the present disclosure, there is provided a device for identifying an iris. As shown in Fig. 5, the device may include: a first shooting component 51, configured to shoot an eye site to obtain an image including the eye site; a processing chip 52, configured to subject the image to image identification to determine an eyeball position parameter in the image and determine a shooting parameter for shooting an iris based on the eye position parameter in the image; and a second shooting component 53, configured to shoot the iris based on the shooting parameter to obtain the iris image and transmit the iris image to the processing chip 52, wherein the processing chip 52 is further configured to identify the iris image to obtain iris feature information and determine whether the iris feature information is matched with predetermined iris feature information.

In an embodiment, the first shooting component 51 is a visible light camera; and the second shooting component 53 is a near-infrared camera.

In an embodiment, the first shooting component 51 and the second shooting component 53 may be the same near-infrared camera.

According to a fourth aspect of embodiments of the present disclosure, there is provided a device for acquiring an iris image. The device may include: a processor; and a memory for storing an instruction executable by the processor, wherein the processor is configured to acquire an image including an eye site; determine an eye position parameter in the image by subjecting the image to image identification; determine a shooting parameter for shooting an iris based on the eye position parameter in the image; and shoot the iris based on the shooting parameter, to obtain the iris image.

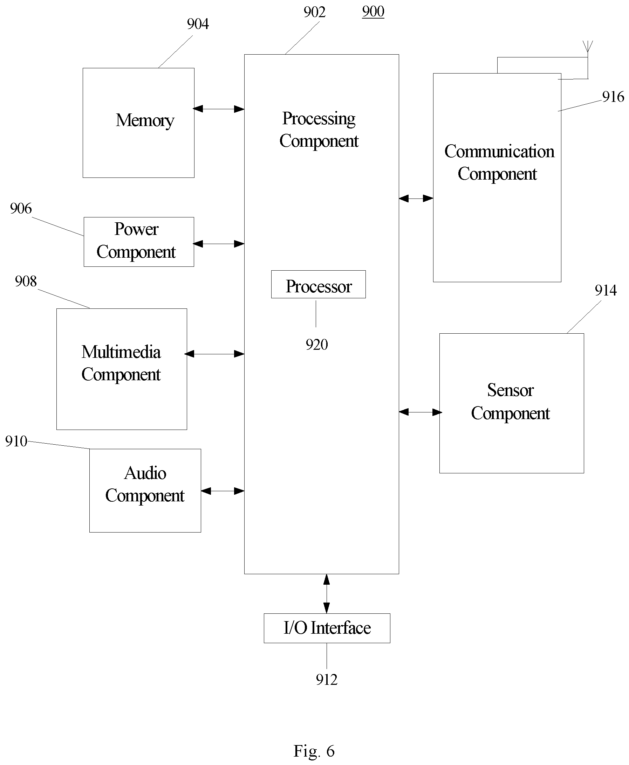

As shown in Fig. 6, there is provided a device 900 for acquiring an iris image according to an illustrative embodiment. The device 900 may be a mobile phone, a computer, a digital broadcast terminal, a message receiving and sending device, a gaming console, a tablet, a medical device, exercise equipment, a personal digital assistant, a vehicle mobile terminal, and the like.

Referring to Fig. 6, the device 900 may include one or more of the following components: a processing component 902, a memory 904, a power component 906, a multimedia component 908, an audio component 910, an input/output (I/O) interface 912, a sensor component 914, and a communication component 916.

The processing component 902 typically controls overall operations of the device 900, such as the operations associated with display, telephone calls, data communications, camera operations, and recording operations. The processing component 902 may include one or more processors 920 to execute instructions to perform all or part of the steps in the above described methods. Moreover, the processing component 902 may include one or more modules which facilitate the interaction between the processing component 902 and other components. For instance, the processing component 902 may include a multimedia module to facilitate the interaction between the multimedia component 908 and the processing component 902.

The memory 904 is configured to store various types of data to support the operation of the device 900. Examples of such data include instructions for any applications or methods operated on the device 900, contact data, phonebook data, messages, pictures, video, etc. The memory 904 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 906 provides power to various components of the device 900. The power component 906 may include a power management system, one or more power sources, and any other components associated with the generation, management, and distribution of power in the device 900.

The multimedia component 908 includes a screen providing an output interface between the device 900 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 908 includes a front camera and/or a rear camera. The front camera and/or the rear camera may receive an external multimedia datum while the device 900 is in an operation mode, such as a photographing mode or a video mode. Each of the front camera and the rear camera may be a fixed optical lens system or have focus and optical zoom capability.

The audio component 910 is configured to output and/or input audio signals. For example, the audio component 910 includes a microphone ("MIC") configured to receive an external audio signal when the device 900 is in an operation mode, such as a call mode, a recording mode, and a voice recognition mode. The received audio signal may be further stored in the memory 904 or transmitted via the communication component 916. In some embodiments, the audio component 910 further includes a speaker to output audio signals.

The I/O interface 912 provides an interface between the processing component 902 and peripheral interface modules, such as a keyboard, a click wheel, buttons, and the like. The buttons may include, but are not limited to, a home button, a volume button, a starting button, and a locking button.

The sensor component 914 includes one or more sensors to provide status assessments of various aspects of the device 900. For instance, the sensor component 914 may detect an open/closed status of the device 900, relative positioning of components, e.g., the display and the keypad, of the device 900, a change in position of the device 900 or a component of the device 900, a presence or absence of user contact with the device 900, an orientation or an acceleration/deceleration of the device 900, and a change in temperature of the device 900. The sensor component 914 may include a proximity sensor configured to detect the presence of nearby objects without any physical contact. The sensor component 914 may also include a light sensor, such as a CMOS or CCD image sensor, for use in imaging applications. In some embodiments, the sensor component 814 may also include an accelerometer sensor, a gyroscope sensor, a magnetic sensor, a pressure sensor, or a temperature sensor.

The communication component 916 is configured to facilitate communication, wired or wirelessly, between the device 900 and other devices. The device 900 can access a wireless network based on a communication standard, such as WiFi, 2G, or 3G, or a combination thereof. In one illustrative embodiment, the communication component 916 receives a broadcast signal or broadcast associated information from an external broadcast management system via a broadcast channel. In one illustrative embodiment, the communication component 916 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In illustrative embodiments, the device 900 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods for acquiring the iris image.

In illustrative embodiments, there is also provided a non-transitory computer-readable storage medium including instructions, such as included in the memory 904, executable by the processor 920 in the device 900, for performing the above-described methods. For example, the non-transitory computer-readable storage medium may be a ROM, a RAM, a CD-ROM, a magnetic tape, a floppy disc, an optical data storage device, and the like.

Other embodiments of the disclosure will be apparent to those skilled in the art from consideration of the specification and practice of the disclosure disclosed here. This application is intended to cover any variations, uses, or adaptations of the disclosure following the general principles thereof and including such departures from the present disclosure as come within known or customary practice in the art, within the scope of the independent claims.

## Claims

1. A method for acquiring an iris image, performed by a mobile phone having an image acquisition function, the method comprising:
shooting (S101) a first image including an eye site, wherein the eye site is a region where an eye is;
determining (S102) an eye position parameter in the first image by subjecting the image to image identification;
determining (S103) a shooting parameter for shooting an iris, based on the eye position parameter in the image; and
shooting (S104) the iris based on the shooting parameter, to obtain a second image that is the iris image,
wherein determining an eye position parameter in the image by subjecting the image to image identification comprises:
determining an eye position in the image by subjecting the image to image identification; and
determining an eye central position and an eye radius in the image based on the eye position using radial symmetry transform (RST) within the eye site.

2. The method according to claim 1, wherein determining an eye position in the image comprises using a self-adaptive AdaBoost algorithm.

3. The method according to any preceding claim, wherein
determining a shooting parameter for shooting an iris based on the eye position parameter in the image comprises: determining a near-infrared shooting parameter for shooting the iris based on the eye position parameter in the image; and
shooting the iris based on the shooting parameter to obtain the iris image comprises:
shooting the iris based on the near-infrared shooting parameter to obtain the iris image.

4. A mobile phone for acquiring an iris image, comprising:
a first acquiring module (21), configured to shoot a first image including an eye site, wherein the eye site is a region where an eye is;
a first determining module (22), configured to determine an eye position parameter in the image by subjecting the first image to image identification;
a second determining module (23), configured to determine a shooting parameter for shooting an iris based on the eye position parameter in the image; and
a second acquiring module (24), configured to shoot the iris based on the shooting parameter, to obtain a second image that is the iris image,
wherein the first determining module comprises:
a first determining sub-module (221), configured to determine an eye position in the image by subjecting the image to the image identification; and
a second determining sub-module (222), configured to determine an eye central position and an eye radius in the image based on the eye position using radial symmetry transform (RST) within the eye site.

5. The mobile phone according to claim 4, wherein the first determining sub-module is configured to determine an eye position in the image using a self-adaptive AdaBoost algorithm.

6. The mobile phone according to any of claims 4 to 5, wherein
the second determining module comprises: a third determining sub-module (232), configured to determine a near-infrared shooting parameter for shooting the iris based on the eye position parameter in the image; and
the second acquiring module comprises: a shooting sub-module, configured to shoot the iris based on the near-infrared shooting parameter to obtain the iris image.

## Patentansprüche

1. Verfahren zum Erfassen eines Irisbilds, durchgeführt mit einem Mobiltelefon mit einer Bilderfassungsfunktion, wobei das Verfahren aufweist:
Aufnehmen (S101) eines ersten Bilds mit einer Augenpartie, wobei die Augenpartie eine Region ist, in der sich ein Auge befindet;
Bestimmen (S102) eines Augenpositionsparameters in dem ersten Bild, indem das Bild einer Bildidentifizierung unterzogen wird;
Bestimmen (S103) eines Aufnahmeparameters zum Aufnehmen einer Iris auf Basis des Augenpositionsparameters in dem Bild; und
Aufnehmen (S104) der Iris auf Basis des Aufnahmeparameters zum Erhalten eines zweiten Bilds, das das Irisbild ist,
wobei das Bestimmen eines Augenpositionsparameters in dem Bild, indem das Bild einer Bildidentifizierung unterzogen wird, aufweist:
Bestimmen einer Augenposition in dem Bild, indem das Bild einer Bildidentifizierung unterzogen wird; und
Bestimmen einer zentralen Augenposition und eines Augenradius in dem Bild auf Basis der Augenposition unter Verwendung von Radialsymmetrietransformation (RST) innerhalb der Augenpartie.

2. Verfahren nach Anspruch 1, wobei das Bestimmen einer Augenposition in dem Bild das Verwenden eines selbstadaptiven AdaBoost-Algorithmus aufweist.

3. Verfahren nach einem der vorhergehenden Ansprüche, wobei
das Bestimmen eines Aufnahmeparameters zum Aufnehmen einer Iris auf Basis des Augenpositionsparameters in dem Bild aufweist: Bestimmen eines Nahinfrarot-Aufnahmeparameters zum Aufnehmen der Iris auf Basis des Augenpositionsparameters in dem Bild; und
Aufnehmen der Iris auf Basis des Aufnahmeparameters zum Erhalten des Irisbilds aufweist:
Aufnehmen der Iris auf Basis des Nahinfrarot-Aufnahmeparameters zum Erhalten des Irisbilds.

4. Mobiltelefon zum Erfassen eines Irisbilds, das aufweist:
ein erstes Erfassungsmodul (21), das zum Aufnehmen eines ersten Bilds mit einer Augenpartie konfiguriert ist, wobei die Augenpartie eine Region ist, in der sich ein Auge befindet;
ein erstes Bestimmungsmodul (22), das zum Bestimmen eines Augenpositionsparameters in dem Bild, indem das erste Bild einer Bildidentifizierung unterzogen wird, konfiguriert ist;
ein zweites Bestimmungsmodul (23), das zum Bestimmen eines Aufnahmeparameters zum Aufnehmen einer Iris auf Basis des Augenpositionsparameters in dem Bild konfiguriert ist; und
ein zweites Erfassungsmodul (24), das zum Aufnehmen der Iris auf Basis des Aufnahmeparameters zum Erhalten eines zweiten Bilds, das das Irisbild ist, konfiguriert ist,
wobei das erste Bestimmungsmodul aufweist:
ein erstes Bestimmungsuntermodul (221), das zum Bestimmen einer Augenposition in dem Bild, indem das Bild der Bildidentifizierung unterzogen wird, konfiguriert ist; und
ein zweites Bestimmungsuntermodul (222), das zum Bestimmen einer zentralen Augenposition und eines Augenradius in dem Bild auf Basis der Augenposition unter Verwendung von Radialsymmetrietransformation (RST) innerhalb der Augenpartie konfiguriert ist.

5. Mobiltelefon nach Anspruch 4, wobei das erste Bestimmungsuntermodul zum Bestimmen einer Augenposition in dem Bild unter Verwendung eines selbstadaptiven AdaBoost-Algorithmus konfiguriert ist.

6. Mobiltelefon nach einem der Ansprüche 4 bis 5, wobei
das zweite Bestimmungsmodul aufweist: ein drittes Bestimmungsuntermodul (232), das zum Bestimmen eines Nahinfrarot-Aufnahmeparameters zum Aufnehmen der Iris auf Basis des Augenpositionsparameters in dem Bild konfiguriert ist; und
das zweite Erfassungsmodul aufweist: ein Aufnahmeuntermodul, das zum Aufnehmen der Iris auf Basis des Nahinfrarot-Aufnahmeparameters zum Erhalten des Irisbilds konfiguriert ist.

## Revendications

1. Procédé d'acquisition d'une image de l'iris, effectué par un téléphone mobile ayant une fonction d'acquisition d'image, le procédé comprenant :
prendre (S101) une première image comprenant un site d'oeil, dans lequel le site d'oeil est une région où se trouve un oeil ;
déterminer (S102) un paramètre de position de l'oeil dans la première image en soumettant l'image à une identification d'image ;
déterminer (S 103) un paramètre de prise de vue pour photographier une iris, sur la base du paramètre de position de l'oeil dans l'image ; et
photographier (S104) l'iris sur la base du paramètre de prise de vue, afin d'obtenir une deuxième image qui est l'image de l'iris,
dans lequel déterminer un paramètre de position de l'oeil dans l'image en soumettant l'image à une identification d'image comprend :
déterminer une position de l'oeil dans l'image en soumettant l'image à une identification d'image ; et
déterminer une position centrale de l'oeil et un rayon de l'oeil dans l'image sur la base de la position de l'oeil en utilisant une transformée de symétrie radiale (RST) dans le site de l'œil.

2. Procédé selon la revendication 1, dans lequel déterminer une position de l'oeil dans l'image comprend utiliser un algorithme AdaBoost auto-adaptatif.

3. Procédé selon l'une quelconque des revendications précédentes, dans lequel
déterminer un paramètre de prise de vue pour photographier une iris sur la base du paramètre de position de l'oeil dans l'image comprend : déterminer un paramètre de prise de vue dans l'infrarouge proche pour photographier l'iris sur la base du paramètre de position de l'oeil dans l'image ; et
photographier l'iris sur la base du paramètre de prise de vue afin d'obtenir l'image de l'iris comprend :
photographier l'iris sur la base du paramètre de prise de vue dans l'infrarouge proche afin d'obtenir l'image de l'iris.

4. Téléphone mobile d'acquisition d'une image de l'iris, comprenant :
un premier module d'acquisition (21), configuré pour prendre une première image comprenant un site d'un oeil, où le site de l'oeil est une région où se trouve l'oeil ;
un premier module de détermination (22), configuré pour déterminer un paramètre de position de l'oeil dans l'image en soumettant la première image à une identification d'image ;
un deuxième module de détermination (23), configuré pour déterminer un paramètre de prise de vue pour photographier une iris sur la base du paramètre de position de l'oeil dans l'image ; et
un deuxième module d'acquisition (24), configuré pour photographier l'iris sur la base du paramètre de prise de vue, afin d'obtenir une deuxième image qui est l'image de l'iris,
dans lequel le premier module de détermination comprend :
un premier sous-module de détermination (221), configuré pour déterminer une position de l'oeil dans l'image en soumettant l'image à l'identification d'image ; et
un deuxième sous-module de détermination (222), configuré pour déterminer une position centrale de l'oeil et un rayon de l'oeil dans l'image sur la base de la position de l'oeil en utilisant une transformée de symétrie radiale (RST) dans le site de l'oeil.

5. Téléphone mobile selon la revendication 4, dans lequel le premier sous-module de détermination est configuré pour déterminer une position de l'oeil dans l'image en utilisant un algorithme AdaBoost auto-adaptatif.

6. Téléphone mobile selon l'une quelconque des revendications 4 à 5, dans lequel
le deuxième module de détermination comprend un troisième sous-module de détermination (232), configuré pour déterminer un paramètre de prise de vue dans l'infrarouge proche pour photographier l'iris sur la base du paramètre de position de l'oeil dans l'image ; et
le deuxième module d'acquisition comprend : un sous-module de prise de vue, configuré pour photographier l'iris sur la base du paramètre de prise de vue dans l'infrarouge proche afin d'obtenir l'image de l'iris.
